**Europäisches Patentamt**

**European Patent Office**

**Office européen des brevets**

(19)

(11) Numéro de publication : **0 501 889 A2**

# (12) DEMANDE DE BREVET EUROPEEN

(21) Numéro de dépôt : **92400513.5**

(22) Date de dépôt : **27.02.92**

(51) Int. Cl.$^5$ : **A61K 31/335,** // (A61K31/415, 31:335), (A61K31/55, 31:335), (A61K31/335, 31:19)

(30) Priorité : **01.03.91 US 662825**

(43) Date de publication de la demande : **02.09.92 Bulletin 92/36**

(84) Etats contractants désignés : **BE CH DE FR GB IT LI NL**

(71) Demandeur : **Laboratoires BIOCODEX**
**19 rue Barbès**
**F-92126 Montrouge Cédex (FR)**

(72) Inventeur : **Lockard, Joan S.**
**14857 S.E. 50th Street**
**Bellevue, Washington 98006 (US)**
Inventeur : **Finnell, Richard H.**
**NE 635 Maple Street**
**Pullman,Washington 99163 (US)**
Inventeur : **Hublot, Bernard M.**
**3 rue des Pommerelles**
**F-60200 Compiegne (FR)**
Inventeur : **Tor, Jacques A.**
**20 rue du Rhône**
**F-60157 Elincourt-Sainte-Marguerite (FR)**
Inventeur : **Les autres inventeurs ont renoncé à leur désignation**

(74) Mandataire : **Varady, Peter et al**
**Cabinet Lavoix 2, Place d'Estienne d'Orves**
**F-75441 Paris Cedex 09 (FR)**

(54) **Composition anti-épileptique comprenant du stiripentol en association avec un médicament anti-épileptique.**

(57) On décrit une composition anticonvulsivante ou anti-épileptique contenant en association ou séparément une quantité thérapeutiquement efficace de stiripentol et une quantité thérapeutiquement efficace d'un composé actif anticonvulsivant ou anti-épileptique, choisi parmi la phénytoïne, la carbamazépine, l'acide valproïque et le valproate de sodium, et son utilisation pour la fabrication d'un médicament anti-épileptique, dans lequel sont atténués ou évités les effets secondaires de composés actifs pour un traitement anti-épileptique.

**EP 0 501 889 A2**

Les patients atteints d'épilepsie ont eu à faire face à trois obstacles successifs avant d'atteindre leur état médical actuel, qui est raisonnablement confortable pour la majorité d'entre eux. Le premier était que les crises épileptiques n'étaient pas considérées comme une maladie mais comme un phénomène surnaturel inspirant de la crainte. Il a fallu plusieurs siècles pour que l'épilepsie soit incluse dans le domaine médical, et des évaluations récentes des problèmes sociologiques rencontrés par des patients épileptiques montrent que certaines difficultés persistent encore.

La seconde étape a été de découvrir un traitement. En les termes de C.B. Radcliffe, "Tout épileptique devrait se souvenir avec gratitude du nom de Sir Charles Locock" [C.B. Radcliffe - *Epilepsy and other convulsive affections of the nervous system. Their pathology and treatment* (L'épilepsie et autres affections convulsives du système nerveux. Leur pathologie et leur traitement), Londres, J. Churchill, 1881], car il a proposé l'emploi de bromures en 1857, avec succès mais au prix d'effets secondaires considérables.

Cela devint le troisième handicap d'une maladie dûment reconnue comme telle, avec un bon nombre de remèdes actifs, à savoir l'acide valproïque, la phénytoïne et la carbamazépine, aucun d'entre eux n'étant dépourvu d'effets secondaires parfois douloureux, au point d'inclure presque le symptôme iatrogénique, la "viscosité mentale" ou "glischroïdie" dans les descriptions de ses patients dûment barbiturisés.

L'un des plus gênants de ces effets indésirables est le "risque de malformations majeures, anomalies mineures et troubles de développement chez le foetus ou le nourrisson" de mères épileptiques. De là, les "Guidelines for the Care of Epileptic Women of Childbearing Age, of the Commission on Genetics, Pregnancy, and the Child, of the International League Against Epilepsy" (Lignes directrices pour les précautions à prendre pour les femmes épileptiques en période de procréation, de la Commission concernant la génétique, la gestation et l'enfant, de la Ligue Internationale contre l'Epilepsie) (*Epilepsia*, 1989, 30, 409-410).

La présente invention concerne l'utilisation du stiripentol pour combattre un tel risque.

Le stiripentol (INN) ou 1-(3,4-méthylènedioxyphényl)-4,4-diméthylpent-1-ène-3-ol, a été décrit dans US-A-3 910 959 (Vallet) en tant que médicament agissant sur le système nerveux central, et en outre a été proposé pour le traitement de l'épilepsie (P. Loiseau et B. Duché dans *Antiepileptic Drugs*, 3e édition, édité par R. Levy et coll., Raven Press, New York, 1989; J.C. Vincent dans *New Anti-convulsant Drugs,* édité par B.S. Meldrum et R.J. Porter, John Libbey, Londres, 1986).

On a découvert à présent que le stiripentol atténue ou évite certains effets et phénomènes indésirables majeurs provoqués par les médicaments anti-épileptiques usuels. Par exemple, alors que tous les médicaments anti-épileptiques majeurs sont des agents tératogènes ou protératogènes [*Teratogenicity of Antiepileptic Drugs: Analysis of Possible Risk Factors* (Tératogénicité de médicaments anti-épileptiques: analyse de facteurs de risques possibles) par Kaneko et coll., *Epilepsia, 29*: 459-467 (1988); *Teratogenicity of Antiepileptic Drug Combinations with Special Emphasis on Epoxide (of Carbamazepine)* (Tératogénicité d'associations de médicaments anti-épileptiques, avec accent particulier sur l'époxyde de carbamazépine) par Lindhout et coll., *Epilepsia, 25*:77-83 (1984); *Fetal Hydantoin Syndrom* (Syndrome foetal de l'hydantoine) par James W.M. Hanson, *Teratology, 13*:185-188, 1976], le stiripentol est un médicament anti-épileptique capable de diminuer ou d'éliminer la tératogénicité associée à ces médicaments.

Un objet de l'invention est l'utilisation du stiripentol éventuellement en association avec un anti-épileptique, pour la fabrication d'un médicament, afin d'atténuer ou d'éviter les effets secondaires des composés actifs utilisés dans ledit traitement de l'épilepsie chez l'homme.

Un objet plus spécifique de l'invention est l'utilisation du stiripentol éventuellement en association avec un anti-épileptique, pour la fabrication d'un médicament afin de lutter contre les effets tératogènes des composés actifs utilisés dans un traitement anti-épileptique de patientes.

Le stiripentol est particulièrement utile dans le traitement de patients recevant de la phénytoïne, de la carbamazépine, de l'acide valproique, du valproate de sodium et similaires, en particulier de la phénytoïne.

Le stiripentol peut être administré à une dose journalière de 5-100 mg/kg, en particulier de 10-50 mg/kg, à savoir par voie orale.

Un autre objet de l'invention est une composition anticonvulsivante ou anti-épileptique contenant, en association ou séparément, une quantité thérapeutiquement efficace de stiripentol et une quantité thérapeutiquement efficace d'un composé actif anticonvulsivant ou anti-épileptique. La composition est de préférence sous une forme appropriée à l'administration orale, à savoir comprimés, gélules, dragées ou sirops.

Une composition préférée contient une dose unitaire orale de 100-500 mg de stiripentol.

Une autre composition préférée est sous la forme de doses unitaires orales contenant 100-500 mg de stiripentol et 100 mg de phénytoïne.

Une autre composition préférée est sous la forme de doses unitaires orales de 100-500 mg de stiripentol et 200-500 mg d'acide valproique ou de valproate de sodium.

Encore une autre composition préférée est sous la forme de doses unitaires orales de 100-500 mg de stiripentol et 100-400 mg de carbamazépine.

Les exemples ci-après sont donnés pour illustrer l'invention.

**EXEMPLE 1:** RESUME D'ETUDES TERATOLOGIQUES: phénytoïne et stiripentol

I- Matériels et méthodes

On a administré régulièrement les composés d'essai, pendant 2 semaines avant les premières tentatives d'accouplement, à trois lignées très pures de souris (SWV, LM/Bc et C57BL/6J). Le stiripentol a été mélangé avec l'aliment moulu pour rongeurs et re-granulé pour être administré dans la nourriture des animaux, tandis que la phénytoïne a été administrée dans l'eau de boisson des animaux. Au cours de ce prétraitement de 2 semaines, on a prélevé du sang sur les animaux par le sinus rétro-orbital, aux jours 7 et 14, et on a déterminé les concentrations de médicament dans le plasma, pour garantir que les mères étaient toutes dans l'éventail thérapeutique souhaité du composé d'essai. Les groupes de traitement pour cette étude sont représentés dans le tableau 1. Chaque groupe de traitement consistait en 10 mères provenant de chacune des trois lignées de souris expérimentales. Les mères ont continué à recevoir le traitement d'essai pendant toute la gestation. Au 18e jour de la gestation, on a sacrifié les mères et on a retiré les foetus. On a noté l'emplacement et la position de tous les foetus viables et de tous les sites de résorption. Les foetus ne présentant aucun mouvement spontané ou provoqué ont été considérés comme morts et inclus parmi les résorption pour les analyses statistiques. On a pesé les foetus viables, on a noté leurs sexes et on les a examinés en ce qui concerne d'importantes malformations externes. Après euthanasie, on a placé au hasard un tiers des foetus dans de l'alcool à 95 % pour coloration du squelette au rouge d'alizarine, et on a placé les foetus restants dans du fixatif de Bouin, en préparation de l'examen de malformations internes à l'aide de la technique à la lame de rasoir de Wilson. Le seuil de signification de 0,05 a été choisi pour toutes les analyses statistiques. Les différences moyennes entre les mesures ont été testées au moyen à la fois d'analyse à double entrée et d'analyse à triple entrée de la variance, après transformation arc-sinus des données lorsque cela était approprié. Lorsque les différences se sont révélées significatives, on a effectué un test de comparaison par paires de Tukey. Le stiripentol et la phénytoïne ont été dosés par HPLC.

II- Groupes de traitement expérimentaux

L'étude a été effectuée en deux parties qui sont désignées par l'année I et l'année II (dans le tableau 2 et dans le texte). Les groupes 1 à 6 ont été inclus dans l'année I et l'année II, tandis qu'on a ajouté le groupe 7 dans l'année II pour déterminer si le traitement simultané par le stiripentol était protecteur contre la tératogénicité associée à une forte dose de phénytoïne (60 mg/kg).

```
                        Tableau 1
Groupe 1   stiripentol (200 mg/kg)
Groupe 2   groupe témoin du groupe 1
Groupe 3   phénytoïne (30 mg/kg)
Groupe 4   phénytoïne (60 mg/kg)
Groupe 5   témoin: eau à pH 10,3
Groupe 6   stiripentol (200 mg/kg) + phénytoïne
           (30 mg/kg)
Groupe 7   stiripentol (200 mg/kg) + phénytoïne
           (60 mg/kg).
```

III- Résultats

1. Tératogénicité de la phénytoïne

Le premier but de l'étude était de déterminer si l'administration simultanée de stiripentol avec la phénytoïne pouvait réduire les risques génétiques (tératogénicité) inhérents à la monothérapie par la phénytoïne. Les résultats donnés dans le tableau 2 représentent le pourcentage d'anomalies foetales totales, malformations à la fois du squelette et des tissus mous, observées dans la descendance de mères affectées à chacun des groupes d'essai. Dans l'année I, la phénytoïne a provoqué des augmentations statistiquement significatives des taux de malformations dans les foetus SWV et C57 aux deux doses (P < 0,05). Un accroissement significatif des malformations n'a été observé dans la lignée LM/Bc qu'à la dose élevée dans l'année I (P < 0,05). Dans l'année II, la phénytoïne a provoqué une augmentation significative d'anomalies congénitales dans les trois lignées à la dose élevée (60 mg/kg), par comparaison aux témoins (P < 0,05). Dans l'année II, la basse concentration de phénytoïne (30 mg/kg) n'a provoqué une augmentation statistiquement significative des malformations que chez les foetus C57 (P < 0,05).

2. Groupes témoins et groupe du stiripentol

Il n'y a pas eu de différences significatives des taux de malformations des groupes 1, 2 et 5, ce qui indique que l'administration orale régulière de stiripentol n'était pas tératogène (P > 0,05). Ces groupes témoins ne pouvaient pas être distingués les uns des autres à la fois dans l'année I et dans l'année II, et par conséquent ces résultats ne sont pas inclus dans le tableau 2.

3. Réduction de la tératogénicité de la phénytoïne par administrations simultanées de stiripentol

(I) Dans l'année I, on a observé une réduction statistiquement significative d'anomalies foetales dans le groupe ayant reçu un traitement de stiripentol/phénytoïne associés, par comparaison avec les groupes ayant reçu la monothérapie par la phénytoïne (30 mg/kg), à la fois dans les lignées SWV et C57 (P < 0,05). Aucune protection n'a pu être déterminée dans la lignée LM/Bc, car la dose de phénytoïne (30 mg/kg) n'était pas tératogène dans cette lignée.

(II) Dans l'année II, le même effet protecteur n'a pu être déterminé statistiquement que pour la lignée C57 (P < 0,05). Là encore, l'absence de tératogénicité significative observée à la dose de phénytoïne (30 mg/kg) dans les lignées LM/Bc et SWV a empêché toute détermination d'un effet protecteur du traitement simultané par le stiripentol.

(III) On a ajouté un septième groupe de traitement dans l'année II, les mères recevant de la phénytoïne (60 mg/kg) conjointement au stiripentol (200 mg/kg), pour déterminer si le traitement simultané par le stiripentol était ou non protecteur avec une forte dose de phénytoïne (60 mg/kg). Ce traitement associé a sensiblement réduit l'incidence de malformations foetales dans les lignées de souris SWV et LM/Bc (P < 0,05).

4. Autres paramètres maternels et foetaux

On a mesuré un certain nombre de paramètres maternels et foetaux, y compris le nombre d'implantations, le nombre de résorptions, le poids foetal moyen et les concentrations de médicament dans le plasma. A la fois dans les années I et II, il n'y a pas eu de différences significatives entre les groupes de traitement, ni dans le nombre de sites d'implantations, ni dans le taux de résorption des produits de conception, dans chaque lignée, indépendamment du traitement médicamenteux (P > 0,05). Dans l'année I, l'exposition intra-utérine à la phénytoïne a eu un effet sensible sur la croissance des foetus, étant donné que des différences significatives de poids des foetus ont été observées dans la lignée SWV aux deux doses de la phénytoïne, et chez les souris C57 au traitement par 60 mg/kg (P < 0,05). Aucune différence significative de poids des foetus n'a été observée dans la lignée LM/Bc dans l'année I. Aucune différence significative des poids moyens des foetus n'a été observée dans aucune lignée dans l'année II de l'étude (P > 0,05).

Tableau 2

Réduction de la tératogénicité de la phénytoïne
par administration simultanée de stiripentol

| Traitement | | SWV | | LM/Bc | | C57BL/6J | |
|---|---|---|---|---|---|---|---|
| Année | | I | II | I | II | I | II |
| PHT (60) % An[1] | | 46,1 | 43,1 | 30,4 | 29,4 | 55,4 | 34,7 |
| | μg/ml | 15,5 | 12,6 | 13,2 | 9,9 | 13,1 | 7,1 |
| PHT (30) % An[1] | | 33,0 | 15,1 | 7,5 | 12,9 | 36,5 | 23,4 |
| | μg/ml | 4,2 | 4,6 | 8,0 | 3,7 | 6,6 | 5,1 |
| PHT (30) STP % An[1] | | 8,7 | 8,8 | 7,4 | 8,8 | 13,0 | 13,3 |
| | μg/ml | 5,6 | 5,3 | 5,9 | 3,8 | 5,5 | 4,0 |
| PHT (60) STP % An[1] | | –[2] | 16,7 | –[2] | 13,7 | –[2] | 12,3 |

[1] % An = pourcentage d'anomalie.

[2] Ce groupe n'a pas été inclu dans l'année I.

Ces études montrent que le stiripentol réduit de façon significative la tératogénicité d'un médicament anti-épileptique classique tel que la phénytoïne.

EXEMPLE 2

Gélules contenant:

stiripentol............................ 500 mg

stéarate de magnésium.................. 3 mg

EXEMPLE 3

Gélules contenant:

stiripentol............................ 500 mg

phénytoïne............................. 100 mg

stéarate de magnésium.................. 3 mg

Revendications

1. Composition anticonvulsivante ou anti-épileptique contenant en association ou séparément une quantité thérapeutiquement efficace de stiripentol et une quantité thérapeutiquement efficace d'un composé actif anticonvulsivant ou anti-épileptique choisi parmi la phénytoïne, la carbamazépine, l'acide valproïque et le valproate de sodium.

2. Composition selon la revendication 1, dans laquelle le composé actif anticonvulsivant ou antiépileptique est la phénytoïne.

3. Composition selon la revendication 1, sous la forme d'une dose unitaire orale contenant 100-500 mg de stiripentol.

4. Composition selon la revendication 1, sous la forme de doses unitaires orales contenant 100-500 mg de stiripentol et 100 mg de phénytoïne.

5. Composition selon la revendication 1, sous la forme de doses unitaires orales de 100-500 mg de stiripentol et 200-500 mg d'acide valproïque ou de valproate de sodium.

6. Composition selon la revendication 1, sous la forme de doses unitaires orales de 100-500 mg de stiripentol et 100-400 mg de carbamazépine.

7. Utilisation du stiripentol et d'un composé choisi parmi la phénytoïne, la carbamazépine, l'acide valproïque et le valproate de sodium,,pour la fabrication d'un médicament anti-épileptique dans lequel sont atténués ou évités les effets secondaires dudit composé.

8. Utilisation selon la revendication 7, pour la fabrication d'un médicament anti-épileptique, permettant de lutter contre les effets tératogènes de composés actifs pour le traitement anti-épileptique de patientes.